(19) Europäisches Patentamt
European Patent Office
Office européen des brevets

(11) Publication number: **0 196 184 B1**

# (12) EUROPEAN PATENT SPECIFICATION

(45) Date of publication of patent specification: **16.09.92**

(51) Int. Cl.[5]: **C07C 271/02**, C07C 271/06, C07D 213/00, A61K 31/16, A61K 31/33

(21) Application number: **86301895.8**

(22) Date of filing: **14.03.86**

The file contains technical information submitted after the application was filed and not included in this specification

## (54) Aryl derivatives.

(30) Priority: **16.03.85 GB 8506870**
**16.03.85 GB 8506871**
**16.03.85 GB 8506872**
**16.03.85 GB 8506873**
**16.03.85 GB 8506874**
**01.06.85 GB 8513863**
**30.12.85 GB 8531839**

(43) Date of publication of application:
**01.10.86 Bulletin 86/40**

(45) Publication of the grant of the patent:
**16.09.92 Bulletin 92/38**

(84) Designated Contracting States:
**AT BE CH DE FR GB IT LI LU NL SE**

(56) References cited:
**EP-A- 0 053 679**
**EP-A- 0 095 683**
**EP-A- 0 127 726**
**FR-A- 2 447 366**
**GB-A- 1 170 653**
**GB-A- 1 427 114**
**GB-A- 1 444 492**
**US-A- 3 219 428**
**US-A- 3 853 905**

(73) Proprietor: **THE WELLCOME FOUNDATION LIMITED**
**Unicorn House 160 Euston Road**
**London NW1 2BP(GB)**

(72) Inventor: **Kneen, Geoffrey**
**P.O. Box 53 Lane End Road**
**High Wycombe Bucks.(GB)**
Inventor: **Jackson, William Paul**
**Langley Court South Eden Park Road**
**Beckenham Kent(GB)**
Inventor: **Islip, Peter John**
**Langley Court South Eden Park Road**
**Beckenham Kent(GB)**
Inventor: **Wates, Peter John**
**Langley Court South Eden Park Road**
**Beckenham Kent(GB)**

(74) Representative: **Garrett, Michael et al**
**The Wellcome Foundation Limited Group Patents and Agreements Langley Court**
**Beckenham Kent BR3 3BS(GB)**

EP 0 196 184 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

THE JOURNAL OF ORGANIC CHEMISTRY, vol. 47, no. 27, 31st December 1982, pages 5283-5289, American Chemical Society, Washington, DC, US; K. YAMADA et al.: "Novel intramolecular photorearrangement of nitronate anions"

"Il Farmaco" edizione scientifica, vol. 31, no. 7, July 1976, pages 470-477, Pavia, IT; E. BELLASIO et al.: "Synthesis of 1-hydroxy and 1-acyloxypyrrolidin-2-ones"

## Description

The present invention relates to certain compounds which are hydroxamic acid aryl derivatives, to methods of preparing such compounds, compositions containing them and to their use in medicine and in other applications.

A class of agents defined in European patent specification no. EP 0 055 418 are described therein as dual inhibitors of the lipoxygenase and cyclo-oxygenase enzymes of the mammalian arachidonic acid metabolism and were found to exhibit anti-inflammatory and related activities. Other compounds which have been described as lipoxygenase and/or cyclo-oxygenase inhibitors include certain naphthyloxy derivatives (eg as described in US patent specification 3 740 437 or in Proc. Ann. Symp. Inst. Basic Med. Sci, Royal College of Surgeons of England, October 1982, pp 263-274). Compounds described in the latter reference include the compound known as nafazatrom.

We have now found that unexpectedly, subject to the provisos (explicit and implicit) set forth below, the compounds of formula (I) as defined hereinbelow, are particularly advantageous inhibitors of the lipoxygenase and/or cyclo-oxygenase enzymes and have useful medical prophylactic and therapeutic properties, as well as certain non-medical uses.

The definition of formula (I)

$$Ar-(L-Ar')_q-(X)_k-(Y)_pN\begin{matrix} / OR^1 \\ \backslash COR^2 \end{matrix} \qquad (I)$$

is thus:

k,p and q are independently 0 or 1, provided that when k is 1 then p must also be 1;

Ar represents either:

(i) naphthyl, tetrahydronaphthyl or pyridyl, any of which is optionally substituted by one or more substituents independently selected from $C_{1-4}$ alkyl (which may itself optionally be substituted by one or more halogen atoms), $C_{1-4}$ alkoxy, halo, nitro, amino, carboxy, $C_{1-4}$ alkoxycarbonyl and hydroxy, or

(ii) phenyl optionally substituted by one or more substituents independently selected from phenyl (optionally substituted by one or more substituents independently selected from those specified as optional substituents in (i) above) and said optional substituents specified in (i) above;

L is selected from $-(CH_2)_r-$ (where r is 1-4), -O-, $-CH_2O-$, $-CH_2S-$, $-OCH_2-$, -CONH-, -NHCO-, -CO- and $-CH_2NH-$, and,

Ar' represents phenylene, thienylene or pyridylene, any of which may be optionally substituted by one or more substituents independently selected from those specified as optional substituents in definition (i) of Ar;

X represents oxygen, sulphur or carbonyl, provided that at least one atom separates said carbonyl group from any carbonyl group in Q as defined below;

Y is $C_{1-10}$ alkylene or $C_{2-10}$ alkenylene;

$R^1$ is independently selected from hydrogen, $C_{1-4}$ alkyl, groups as defined for Ar above and groups of formula $-COR^3$ in which $R^3$ is selected from $C_{1-4}$ alkyl (optionally substituted by a carboxy or $C_{1-4}$ alkoxycarbonyl group) and groups of formula $-N(R^4)R^5$ in which $R^4$ is hydrogen or $C_{1-4}$ alkyl and $R^5$ represents hydrogen $C_{1-4}$ alkyl or phenyl optionally substituted by one or more substituents independently selected from those specified as optional substituents in the definition (i) of Ar,

and $R^2$ is selected from hydrogen, $C_{1-4}$ alkyl, amino, $C_{1-4}$ alkylamino, di-$C_{1-4}$ alkylamino, $C_{5-7}$ cycloalkylamino, $C_{5-7}$ cycloalkyl ($C_{1-4}$ alkyl) amino, anilino, N-$C_{1-4}$ alkylanilino and groups as defined for Ar above;

and salts thereof;

with the proviso that:-

when q is 0, k is 0 or 1 and p is 1, Ar is phenyl or naphthyl, either being optionally substituted by one or more substituents as specified in definition (i) of Ar, and X is oxygen or sulphur (in the case when k is 1) Y is $C_{1-10}$ alkylene and $R^1$ and $R^2$ is hydrogen or $C_{1-4}$ alkyl;

then the other of $R^1$ and $R^2$ is neither hydrogen nor $C_{1-4}$ alkyl.

The unexpected advantages we have found in compounds of formula (I) and their salts are selected from one or more of the following, namely: surprisingly high potency, surprising oral efficacy, surprising efficacy by inhalation and surprisingly long duration of action.

It should be appreciated that we make no claim to those compounds of formula (I) and their salts, processes for their preparation, compositions containing them and their use, which are not novel having regard to following references:-

a) Patent Specifications:

| | |
|---|---|
| EP 0 161 939 A | |
| GB 1 226 344 | GB 1 427 114 |
| GB 1 278 739 | GB 1 437 783 |
| GB 1 315 830 | GB 1 444 492 |
| GB 1 382 996 | GB 2 047 234 A |
| GB 1 396 726 | |
| US 3 600 437 | US 3 972 934 |
| US 3 821 289 | US 3 978 116 |
| US 3 890 377 | |
| JP 57035543 | JP 57062239 |

b) Literature references:-

Tetrahedron 1970 26 (23) 5653-64

Eur. J. Med. Chem. Chimica. Therapeutica 1975 10 (2) 125-128

Eur. J. Med. Chem. Chimica. Therapeutica 1970 13 (2) 211-13

J. Chem. Eng. Data 1985 30 237-9

Chem. Biol. Hydroxamic Acids [Proc. Int. Symp.] 1981, 51-62

Arzneim. Forsch. 1978 28 (11) 2087-92

Thus for example, European patent specification 0 161 939 A discloses a genus of compounds which are alleged to be anti-allergic and anti-asthmatic inhibitors of delta-5 lipoxygenase. This genus embraces compounds of formula (I) as hereinbefore defined which inter alia, are excluded by the proviso that:-

when n is 0 and m is 1,

when $R^1$ is hydrogen or $C_{1-4}$ alkyl and $R^2$ is phenyl optionally substituted by a single substituent selected from $C_{1-4}$ alkyl, $C_{1-4}$ alkoxy and phenyl optionally substituted by one or more substituents independently selected from these specified as optional substituents in definition (i) of Ar,

then at least one of k and p must be 1, and in the case when k is 0 and p is 1, Y must be $C_{2-10}$ alkenylene.

Throughout this specification, unless indicated to the contrary, alkyl and alkyl-containing moieties (such as alkylene and alkoxy) can be either straight or branched. Alkyl of 1-4 carbon atoms whether alone or part of another moiety comprises methyl, ethyl, n-propyl, iso-propyl, n-butyl, iso-butyl and t-butyl. For use in medicine, the salts of the compounds of formula (I) are those salts which are physiologically acceptable. However, non-physiologically acceptable salts are included within the ambit of the present invention, either for use in non-medical applications such as further described hereinbelow, or as may be used in the preparation of compounds of formula (I) and their physiologically acceptable salts.

When Ar represents optionally-substituted naphthyl, this may represent naphth-1-yl or naphth-2-yl, although the latter is generally preferred.

When Ar represents optionally substituted tetrahydronaphthyl, 5, 6, 7, 8-tetrahydronaphth -1-and -2-yl are preferred, especially -2-yl.

When Ar represents optionally substituted pyridyl, pyrid-2-yl and pyrid-4-yl are preferred.

When q is 1 and Ar' represents optionally substituted phenylene, -L- and $-(X)_k$-are preferably meta- or para-substituted relative to one another on the phenyl ring.

When q is 1 and Ar' represents optionally substituted thienylene, -L- and $-(X)_k$-are preferably attached in the 1- and the 5- positions of the phenylene ring.

When q is 1 and Ar' represents optionally substituted pyridylene, -L- and $-(X)_k$-are preferably attached in the 1- and the 6- positions of the pyridyline ring.

One preferred sub-class of the compounds of formula (I) comprises those compounds wherein

q is 0 and p is 1;

Ar represents naphthyl optionally substituted by one or more substituents independently selected from those defined in definition (i) of Ar;

X (in the case when k is 1) is oxygen; and

$R^2$ is $C_{1-4}$ alkyl;

and salts thereof.

An especially preferred group of compounds and salts within the latter defined sub-class comprises

those wherein $R^1$ is hydrogen and $R^2$ is methyl. This group includes compounds and salts wherein Ar is unsubstituted.

q and p are 1 and k is 0;

Another preferred sub-class of the compounds of formula (I) comprises those compounds wherein

Ar represents phenyl optionally substituted by one or more substituents independently selected from those defined in definition (ii) of Ar;

L is -O- or $-CH_2O-$; and

Ar' is phenylene optionally substituted by one or more substituents independently selected from those defined in definition (i) of Ar; and salts thereof.

An especially preferred group of compounds and salts within the latter defined sub-class comprises those wherein Ar and/or Ar' are substituted only by one or two fluorine atoms or both are unsubstituted, and $R^2$ is $C_{1-4}$ alkyl, particularly methyl. Of this group, meta- or para- relative substitution of L and $-(X)_k-$ on Ar' is preferred.

Included within the general class of the compounds of formula (I) and their salts are the following which we may claim separately, namely those wherein:-

(i) k is 1 and X represents oxygen;
(ii) k is 1 and X represents sulphur;
(iii) q is 0;
(iv) q is 1;
(v) q is 1 and -L- is selected from-O-, $CH_2O-$, $-CH_2S-$, -NHCO-and -CO-;
(vi) Ar' is optionally substituted phenylene;
(vii) Ar' is optionally substituted thienylene;
(viii) Ar' is optionally substituted pyridylene;
(ix) Ar is optionally substituted naphthyl;
(x) Ar is optionally substituted tetrahydronaphthyl;
(xi) Ar is optionally substituted pyridyl;
(xii) k is 0;
(xiii) p is 0;
(xiv) p is 1 and Y is $C_{1-10}$ alkylene;
(xv) p is 1 and Y is $C_{2-10}$ alkenylene;
(xvi) any two or more of (i)-(xv) together in combination, provided the combination is compatible with the provisos contained within formula (I) as hereinbefore defined.

Preferred compounds of formula (I) include N-(3-phenoxycinnamyl)acetohydroxamic acid, N-(4-benzyloxybenzyl)acetohydroxamic acid, N-[2-(5,6,7,8-tetrahydro-2-naphthyloxy) ethyl]acetohydroxamic acid and N-(5,6,7,8-Tetrahydro-2-naphthylallyl)acetohydroxamic acid. Salts of the latter compounds are also preferred.

Examples of other compounds of formula (I) and their salts include the following and salts thereof:

5,6-Dihydro-4-hydroxy-6-(1-naphthyl)-1,4-thiazin-3(2H,4H)-one
1-Hydroxy-5-(4-isobutylphenethyl)-2-pyrrolidone
1-Hydroxy-5-[3-(6-methoxy-2-naphthyl)butyl]-2-pyrrolidone
5,6-Dihydro-4-hydroxy-6-[1-(6-methoxy-2-naphthyl)ethyl]-1,4-thiazin-3-(2H,4H)-one
5-[3-(2-Fluoro-4-biphenylyl)butyl]-1-hydroxy-2-pyrrolidone
6-[1-(2-Fluoro-4-biphenylyl)ethyl]-5,6-dihydro-4-hydroxy-1,4-thiazin-3(2H,4H)-one
1-Hydroxy-5-[3-(4-isobutylphenyl)butyl]-2-pyrrolidone
5,6-Dihydro-4-hydroxy-6-[1-(4-isobutylphenyl)butyl]-1,4-thiazin-3-(2H,4H)-one
N-[1(4-biphenylyl)ethyl]acetohydroxamic acid
N[2-(2-Fluoro-4-biphenylyl)propyl]acetohydroxamic acid
4-(2-Fluoro-4-biphenylyl)-N-methylpentanohydroxamic acid
5-(4-Biphenylyl)-N-methyl-5-oxopentanohydroxamic acid
N-[2-(4-Biphenylyloxy)ethyl]acetohydroxamic acid
N-[2-(4-Biphenylyloxy)ethyl]isobutyrohydroxamic acid
N-[3-(4-Biphenylyloxy)propyl]acetohydroxamic acid
2-(4-Biphenylyloxy)-N-methylacetohydroxamic acid
3-(4-Biphenylyloxy)-N-methylpropionohydroxamic acid
7-(4-Biphenylyloxy)-N-methylheptanohydroxamic acid
3-(4-Biphenylyl)-N-methylpropionohydroxamic acid
5-(4-Biphenylyl)-N-methylpentanohydroxamic acid
N-Methyl-2-(5,6,7,8-tetrahydro-1-(or 2-)naphthoxy)acetohydroxamic acid

N-Methyl-3-(5,6,7,8-tetrahydro-1-(or 2-)naphthoxy)propionohydroxamic acid
N-Methyl-7-(5,6,7,8-tetrahydro-1-(or 2-)naphthoxy)heptanohydroxamic acid
Methyl N-methyl-3-(5,6,7,8-tetrahydro-1-(or 2-)naphthoxy)propionohydroxamate
N-[3-(5,6,7,8-tetrahydro-1-(or 2-)naphthoxy)propyl]acetohydroxamic acid
N-[6-(5,6,7,8-tetrahydro-1-(or 2-)naphthoxy)hexyl]acetohydroxamic acid
N-[2-(5,6,7,8-tetrahydro-1-(or 2-)naphthoxy)ethyl]isobutyrohydroxamic acid
N-Methyl-3-(5,6,7,8-tetrahydro-1-(or 2)naphthyl)propionohydroxamic acid
N-Methyl-5-(5,6,7,8-tetrahydro-1-(or 2-)naphthyl)pentanohydroxamic acid
N-[3-(5,6,7,8-tetrahydro-1-(or 2-)naphthyl)propyl]acetohydroxamic acid
N-[5-(5,6,7,8-tetrahydro-1-(or 2-)naphthyl)pentyl]acetohydroxamic acid
N-[3-(5,6,7,8-tetrahydro-1-(or 2-)naphthyl)propyl]isobutyrohydroxamic acid
N-[2-(2-naphthyloxy)ethyl]benzohydroxamic acid
2-Benzyloxy-N-methylbenzohydroxamic acid
N-methyl-3-benzyloxybenzohydroxmic acid
4-benzyloxy-N-methylbenzohydroxamic acid
N-methyl-3-(2-naphthyloxymethyl)benzohydroxamic acid
3-(2-biphenylyloxymethyl)-N-methylbenzohydroxamic acid
N-[1-(4-benzyloxy-2-hydroxyphenyl)ethyl]-acetohydroxamic acid
N-(2-benzyloxybenzyl)acetohydroxamic acid,
N-[4-(1-naphthylmethoxy)benzyl]acetohydroxamic acid
N-(3-phenoxybenzyl)acetohydroxamic acid
N-(3-benzyloxybenzyl)acetohydroxamic acid
N-methyl-3-(1-naphthyloxymethyl)benzohydroxamic acid
3-benzylamino-N-methylbenzohydroxamic acid
4-benzylamino-N-methylbenzohydroxamic acid
5-benzyloxy-2-hydroxy-N-methylbenzohydroxamic acid
4-benzyloxy-2-hydroxy-N-methylbenzohydroxamic acid
4-benzamido-N-methylbenzohydroxamic acid
3-benzamido-N-methylbenzohydroxamic acid
3-benzoyl-N-methylbenzohydroxamic acid
5-benzoyloxy-N-methylthiophen-2-carbohydroxamic acid
4-benzoyloxy-N-methylthiophen-2-carbohydroxamic acid
3-(biphenyl-2-yloxymethyl)-N-methylbenzohydroxamic acid
N-(4-Biphenylylmethyl)acetohydroxamic acid
N-[4-(2-Naphthylmethoxy)benzyl]acetohydroxamic acid
N-(4-Phenoxybenzyl)acetohydroxamic acid
N-(4-Benzyloxybenzyl)pivalohydroxamic acid
N-(4-Benzyloxybenzyl)-2-methylpropanohydroxamic acid
N-(4-Phenylcarbamoylbenzyl)acetohydroxamic acid
N-[(2',4'-Difluoro-4-biphenylyl)methyl]acetohydroxamic acid
N-[1-(2',4'-Difluoro-4-biphenylyl)ethyl]-2,2-dimethylpropanohydroxamic acid
N-[4-(4-Biphenylylmethoxy)benzyl]acetohydroxamic acid
N-[4-(2,4-Difluorobenzyloxy)benzyl]acetohydroxamic acid
N-[4-(2,4-Difluorobenzyloxy)benzyl]pivalohydroxamic acid
N-[5,6,7,8-Tetrahydro-2-naphthyl)methyl]acetohydroxamic acid
N-[2-(5,6,7,8-Tetrahydro-2-naphthyloxy)ethyl]-pivalohydroxamic acid
N-(5,6,7,8-Tetrahydro-2-naphthylallyl)pivalohydroxamic acid
N-(5,6,7,8-Tetrahydro-2-naphthylmethyl)pivalohydroxamic acid
N-[2-(2',4'-Difluoro-4-biphenylyl)ethylacetohydroxamic acid
N-(4-Isobutylbenzyl)acetohydroxamic acid
N-[1-(4-Biphenyl)ethyl]pivalohydroxamic acid
N-[(4-Biphenylyl)methyl]pivalohydroxamic acid
5-[2-(4-Benxyloxyphenyl)ethyl]-1-hydroxy-2-pyrrolidone
1-Hydroxy-1-[2-(2-naphthyloxy)ethyl]-3-phenylurea
N-(4-Benzyloxybenzyl)-N-hydroxy-N-methylurea
N-(4-Benzyloxybenzyl)-O-methylcarbamoylacetohydroxamic acid
N-[2-(2-Naphthylthio)ethyl]-N-(phenylcarbamoyloxy)acetamide
N-[4-(Benzyloxybenzyl)-O-(methylcarbamoyl)pivalohydroxamic acid

N-(4-Benzyloxybenzyl)-O-(2,2-dimethylethyl)carbamoylacetohydroxamic acid
N-(4-Benzyloxybenzyl)-N-(t-butylcarbonyloxy)acetamide
3-[N-(4-Benzyloxybenzyl)acetamidooxycarbonyl]propanoic acid
N-[3-(4-Benzyloxyphenyl)prop-2-enyl]acetohydroxamic acid
N-[3-(4-Benzyloxyphenyl)prop-2-enyl]acetohydroxamic acid
N-[4-(2-Pyridyl)benzyl]acetohydroxamic acid
N-[4-(2,4-Difluorophenoxymethyl)benzyl]acetohydroxamic acid
N-[4-(2-Pyridyloxy)benzyl]acetohydroxamic acid
N-(5-Phenoxymethyl-2-thienylmethyl)acetohydroxamic acid
N-(6-Phenoxy-2-pyridylmethyl)acetohydroxamic acid
N-[3-(4-Fluoro-3-phenoxyphenyl)prop-2-enyl]acetohydroxamic acid
N-[4-(Benzylthio)benzyl]acetohydroxamic acid
N-[3-(2-Pyridyloxy)benzyl]acetohydroxamic acid
N-[3-(4-Fluoro-3-phenoxyphenyl)prop-2-enyl]-N-hydroxy-N-methylurea
N-[3-(4-Fluoro-3-phenoxyphenyl)prop-2-enyl]-N-hydroxy-N'-t-butylurea
N-[3-(4-Fluoro-3-phenoxyphenyl)prop-2enyl]-N-hydroxy-N'-cyclohexylurea
N-[3-(4-Fluoro-3-phenoxyphenyl)prop-2-enyl]-N-hydroxy-N'-phenylurea
N-(Biphenyl-4-ylmethyl)-N-hydroxy-N'-methylurea
N-(Biphenyl-4-ylmethyl)-N-hydroxy-N'-t-butylurea
N-(Biphenyl-4-ylmethyl)-N-hydroxy-N'-cyclohexylurea
N-(Biphenyl-4-ylmethyl)-N-hydroxy-N'-phenylurea
N-Hydroxy-N'-methyl-N-[3-(2-pyridyloxy)benzyl]urea
3-(2-Pyridyloxy)prop-2-enohydroxamic acid
1-Hydroxy-5-(2-pyridyl)piperazin-2-one
N-(2-[4-(2-Pyridylmethoxy)phenoxy]ethyl)acetohydroxamic acid
N-(2-[3-(2-Pyridylmethoxy)phenoxy]ethyl)acetohydroxamic acid
N-(2-[4-(Benzyloxy)phenoxy]ethyl)acetohydroxamic acid
N-[4-Fluoro-2-phenoxyphenyl)methyl]-N-hydroxy-N'-methylurea
N-[4-Fluoro-3-phenoxyphenyl)methyl]-N-hydroxy-N'-phenylurea
N-[3-(Biphenyl-4-yl)prop-2-enyl]-N-hydroxy-N'-methylurea
N-[3-(Biphenyl-4-yl)prop-2-enyl]-N-hydroxy-N'-phenylurea
N-[(4-Benzyloxyphenyl)methyl]-N-hydroxyurea
N-[(4-Benzyloxyphenyl)methyl]-N-hydroxy-N'-methylurea
N-[4-(4-hydroxybenzyloxy)benzyl]acetohydroxamic acid
N-[4-(4-pyridylmethoxy)methyl]acetohydroxamic acid
3-[(4-Fluoro-3-phenoxyphenyl)methyl]-1-hydroxy-1,3-dihydroimidazol-2-one
3-[(2-Fluorobiphenyl-4-yl)methyl]-1-hydroxy-1,3-dihydroimidazol-2-one
1-Hydroxy-3-[3-(4-phenoxyphenyl)prop-2-enyl]-1,3-dihydroimidazol-2-one
3-[4-(4-Fluoro-3-phenoxyphenyl)prop-2-enyl]-1-hydroxy-1,3-dihydroimidazol-2-one

Further examples of compounds of formula (I) include each and every specific compound listed below:-
N-Methyl-3-(Ar)acrylohydroxamic acid,
N-Methyl-5-(Ar)-2,4-pentadienohydroxamic acid,
N-Methyl-4-(4-isobutylphenyl)-2-butenohydroxamic acid,
N-[3-(Ar)-2-propenyl]acetohydroxamic acid and
N-[3-(Ar)-2-propenyl]isobutyrohydroxamic acid
wherein Ar represents

i) 1 (or 2)-naphthyl
ii) 4-isobutylphenyl
(iii) 4-biphenylyl
(iv) 2 (or 3 or 4)-benzyloxyphenyl
(v) phenyl or
(vi) 4-methylphenyl

It should be appreciated that any compound in the foregoing list may be claimed alone or with one or more of the others as constituting a preferred aspect of the present invention.

Subject to any limitations expressed implied herein, the present invention also provides any compound of formula (I) (as hereinbefore defined) or physiologically acceptable salt thereof for use as an inhibitor of the lipoxygenase and/or cyclo-oxygenase enzymes of the mammalian arachidonic acid metabolism, to methods of inhibition of such enzyme(s) by administration to a mammal of a lipoxygenase and/or cyclo-oxygenase (as appropriate) inhibiting amount of any such compound or salt, and to use of any such compound or salt in the manufacture of lipoxygenase and/or cyclo-oxygenase inhibitor (as appropriate) agents.

Further, and also subject to any limitations expressed or implied herein, the present invention also provides any compound of formula (I) (as hereinbefore defined) or physiologically acceptable salt thereof, for use as a medical therapeutic and/or prophylactic agent, to methods of medical therapeutic and/or prophylactic treatment by administration to a mammal of a medically therapeutic and/or prophylactic (as appropriate) effective amount of any such compound or salt, and to use of any such compound or salt in the manufacture of medical therapeutic and/or prophylactic (as appropriate) agents. The kinds of medical therapy and prophylaxis pertinent to the foregoing and therefore in that sense comprising part of the present invention, are elaborated by way of example in the following paragraphs which are not intended to be construed as in any way limiting the scope of these aspects of said invention.

By virtue of their lipoxygenase inhibitory properties, said compounds and salts find application in the treatment and/or prophylaxis of any condition where a lipoxygenase inhibitor is indicated, especially spasmogenic and allergic conditions and tumours.

By virtue of their cyclo-oxygenase inhibitory properties, said compounds and salts find application in the treatment and/or prophylaxis of any condition where a cyclo-oxygenase inhibitor is indicated, especially pyresis and pain.

By virtue of both their lipoxygenase and cyclo-oxygenase inhibitory properties, said compounds and salts find application in the treatment and/or prophylaxis of any condition where a dual lipoxygenase/cyclo-oxygenase inhibitor is indicated, especially any condition involving blood platelet aggregation or inflammation. In the case of inflammation, the compounds and salts are particularly suited to the treatment and/or prophylaxis of conditions associated with infiltration of leucocytes into inflamed tissue.

In determining when a lipoxygenase, cyclo-oxygenase or dual lipoxygenase/cyclo-oxygenase inhibitor is indicated, of course inter alia, the particular condition in question and its severity must be taken into consideration and this determination is ultimately at the discretion of the attendant physician.

Examples of the aforesaid spasmogenic conditions are those involving smooth muscle tissue, especially airway smooth muscle constriction such as intrinsic asthma (including intrinsic or idiopathic bronchial asthma and cardiac asthma), bronchitis and arterial smooth muscle constriction such as coronary spasm (including that associated with myocardial infarction, which may or may not lead to left ventricular failure resulting in cardiac asthma) and cerebral spasm or 'stroke'. Other examples include bowel disease caused by abnormal colonic muscular contraction such as may be termed 'irritable bowel syndrome', 'spastic colon' or 'mucous colitis'.

Examples of the aforesaid allergic conditions are extrinsic asthma (from which it will be appreciated that said compounds and salts are particularly favourable as anti-asthmatic agents), allergic skin diseases such as eczema having a total or partial allergic origin, allergic bowel disease (including coeliac disease) and allergic eye conditions such as hayfever (which may additionally or alternatively affect the upper respiratory tract) and allergic conjunctivitis. Examples of the aforesaid tumours are skin neoplasms, both benign and malignant.

Examples of the aforesaid pyretic and painful conditions include fever associated with infections, trauma and injury, malignant disease, and diseases affecting the immune system (including anto-immune diseases).

Examples of the aforesaid conditions involving blood platelet aggregation are those resulting from thrombosis, including 'stroke' having a total or partial thrombotic origin, coronary thrombosis, phlebitis and phlebothrombosis (the latter two conditions also possibly being associated with inflammation).

Examples of the aforesaid conditions involving inflammation are inflammatory conditions of the lung, joints, eye, bowel, skin and heart.

Inflammatory lung conditions which may be so treated and/or prevented include asthma and bronchitis ( vide supra) and cystic fibrosis (which may also or alternatively involve the bowel or other tissue).

Inflammatory joint conditions which may be so treated and/or prevented include rheumatoid arthritis, rheumatoid spondylitis, osteoarthritis, gouty arthritis and other arthritic conditions.

Inflammatory eye conditions which may be so treated and/or prevented include uveitis (including iritis) and conjunctivitis ( vide supra).

Inflammatory bowel conditions which may be so treated and/or prevented include Crohn's disease and ulcerative colitis.

Inflammatory skin diseases which may be so treated and/or prevented include those associated with cell proliferation, such as psoriasis and eczema ( vide supra) and dermatitis (whether or not of allergic origin).

Inflammatory conditions of the heart which may be so treated and/or prevented include coronary infarct

damage.

Other inflammatory conditions which may be so treated and/or prevented include tissue necrosis of chronic inflammation and tissue rejection following transplant surgery.

It is also believed that the compound of formula (I) and their physiologically acceptable salts are effective agents in the prophylaxis and/or treatment of bacterial and fungal infections, this forming a further aspect of the present invention in like manner.

It is known in the literature that some compounds which are cyclo-oxygenase and/or lipoxygenase inhibitors can delay the decay of cut plant matter. Thus, it is now believed that by virtue of their enzyme inhibitory effects, the compounds of formula (I) and salts thereof are also useful for controlling the processes of growth and decay in plants. Thus the present invention also provides the compounds of formula (I) and their salts for use in a method of regulating the growth of, or delaying senescence in vegetable matter by application to said matter of an effective amount of a compound of formula (I) or a salt thereof.

The term senescence refers to the process whereby plant matter decays, especially after being picked, cut or otherwise removed from its normal growing environment. Vegetable matter includes trees, shrubs, flowers and edible vegetables and other food crops.

The above method is particularly applicable to flowers intended for decorative or display purposes such as carnations, crysanthemums, daisies, begonias, etc. These include perennial annual and biannual flowers, for example those that grow from bulbs (eg dahlias) or from seed (eg marigolds). The method is also especially suited to use with decorative shrubs and trees, for example those which are displayed when cut, such as christmas trees.

The compound of formula (I) and their salts may also be used for the preservation of picked fruits.

For medical use, the amount required of a compound of formula (I) or physiologically acceptable salt thereof (hereinafter referred to as the active ingredient) to achieve a therapeutic effect will, of course, vary both with the particular compound, the route of administration and the mammal under treatment and the particular disorder or disease concerned. A suitable dose of a compound of formula (I) or physiologically acceptable salt thereof for a mammal suffering from, or likely to suffer from any condition as described hereinbefore is 0.1 $\mu$g-500mg of base per kilogram bodyweight. In the case of systemic administration, the dose may be in the range 0.5 to 500 mg of base per kilogram bodyweight, the most preferred dosage being 0.5 to 50 mg/kg of mammal bodyweight for example 5 to 25 mg/kg; administered two or three times daily. In the case of topical administration, eg. to the skin or eye, a suitable dose may be in the range 0.1$\mu$g - 100$\mu$g of base per kilogram, typically about 0.1 $\mu$g/Kg.

In the case of oral dosing for the treatment or prophylaxis of airway smooth muscle constriction, or asthma or bronchitis in general, due to any course, a suitable dose of a compound of formula (I) or physiologically acceptable salt thereof, may be as specified in the preceding paragraph, but most preferably is from is 1 mg to 10 mg of base per kilogram, the most preferred dosage being from 1 mg to 5 mg/kg of mammal bodyweight, for example from 1 to 2 mg/kg. In the case of pulmonary administration for the latter indications, the dose may be in the range of from 2 $\mu$g to 100 mg, for example from 20 $\mu$g to 0.5 mg, especially 0.1 to 0.5 mg/kg.

While it is possible for an active ingredient to be administered alone, it is preferable to present it as a pharmaceutical formulation comprising a compound of formula (I) or a pharmacologically acceptable acid addition salt thereof and a physiologically acceptable carrier therefor. Such formulations consitute a further feature of the present invention. Conveniently, the active ingredient comprises from 0.1% to 99.9% by weight of the formulation. Conveniently, unit doses of a formulation contain between 0.1 mg and 1 g of the active ingredient. For topical administration, the active ingredient preferably comprises from 1% to 2% by weight of the formulation but the active ingredient may comprise as much as 10% w/w. Formulations suitable for nasal or buccal administration, (such as self-propelling powder dispensing formulations described hereinafter), may comprise 0.1 to 20% w/w, for example 2% w/w of active ingredient.

The formulations, both for veterinary and for human medical use, of the present invention comprise an active ingredient in association with a pharmaceutically acceptable carrier therefor and optionally other therapeutic ingredient(s). The carrier(s) must be 'acceptable' in the sense of being compatible with the other ingredients of the formulations and not deleterious to the recipient thereof.

The formulations include those in a form suitable for oral, pulmonary, ophthalmic, rectal, parenteral (including subcutaneous, intramuscular and intravenous), intra-articular, topical, nasal or buccal administration.

The formulations may conveniently be presented in unit dosage form and may be prepared by any of the methods well known in the art of pharmacy. All methods include the step of bringing the active ingredient into association with the carrier which constitutes one or more accessory ingredients. In general,

the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units such as capsules, cachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be in the form of a bolus, electuary or paste.

A tablet may be made by compressing or moulding the active ingredient optionally with one or more accessory ingredients. Compressed tablets may be prepared by compressing, in a suitable machine, the active ingredient in a free-flowing form such as a powder or granules, optionally mixed with a binder, lubricant, inert diluent, surface active or dispersing a gent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration conveniently comprise a sterile aqueous preparation of the active ingredient which is preferably isotonic with the blood of the recipient.

Formulations suitable for intra-articular administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Formulations suitable for topical administration include liquid or semi-liquid preparations such as liniments, lotions, applications; oil-in-water or water-in-oil emulsions such as creams, ointments or pastes; or solutions or suspensions such as drops. For example, for ophthalmic administration, the active ingredient may be presented in the form of aqueous eye drops as, for example, a 0.1 - 1.0% solution. Formulations suitable for administration to the nose or buccal cavity include powder, self-propelling and spray formulations such as aerosols and atomizers. The formulations, when dispersed, preferably have a particle size in the range of 0.1 to 200$\mu$.

A particularly valuable form of a pharmaceutical composition of the present invention, for use in the prophylaxis or treatment of airway smooth muscle constriction, or asthma or bronchitis in general, due to any cause, is one suitable for pulmonary administration via the buccal cavity. Preferably the composition is such that particles having a diameter of 0.5 to 7 $\mu$, most preferably 1 to 6 $\mu$, containing active ingredient, are delivered into the lungs of a patient. Such compositions are conveniently in the form of dry powders for administration from a powder inhalation device or self-propelling powder-dispensing containers, for example as a self-propelling aerosol composition in a sealed container; preferably the powders comprise particles containing active ingredient of which particles at least 98% by weight have a diameter greater than 0.5 $\mu$ and at least 95% by number have a diameter less than 7 $\mu$. Most desirably at least 95% by weight of the particles have a diameter greater than 1 $\mu$ at least 90% by number of the particles have a diameter less than 6 $\mu$.

The compositions in the form of dry powders preferably include a solid fine powder diluent such as sugar and are conveniently presented in a pierceable capsule, for example of gelatin.

Self-propelling compositions of the invention may be either powder-dispensing compositions or compositions dispensing the active ingredient in the form of droplets of a solution or suspension. Self-propelling powder-dispensing compositions include a liquid propellant having a boiling point of below 65 °F at atmospheric pressure. Generally the propellant may constitute 50 to 99.9% w/w of the composition whilst the active ingredient may constitute 0.1 to 20% w/w, for example about 2% w/w of the composition. The carrier in such compositions may include other constituents, in particular a liquid non-ionic or solid anionic surfactant, or a solid diluent (preferably having a particle size of the same order as of the particles of active ingredient) or both. The surfactant may constitute from 0.01 up to 20% w/w, though preferably it constitutes below 1% w/w of the composition.

Self-propelling compositions wherein the active ingredient is present in solution comprise an active ingredient, propellant and co-solvent, and advantageously an antioxidant stabiliser. The co-solvents may constitute 5 to 40% w/w of the composition, though preferably less than 20% w/w of the composition.

Compositions of the present invention may also be in the form of aqueous or dilute alcoholic solution, optionally a sterile solution, of the active ingredient for use in a nebuliser or atomiser.

Formulations of the present invention may also be in the form of an aqueous or dilute alcoholic solution, optionally a sterile solution, of the active ingredient for use in a nebuliser or atomiser, wherein an

accelerated air steam is used to produce a fine mist consisting of small droplets of the solution. Such formulations usually contain a flavouring agent such as saccharin sodium and a volatile oil. A buffering agent and a surface active agent may also be included in such a formulation which should also contain a preservative such as methylhydroxybenzoate.

Other formulations suitable for nasal administration include a coarse powder having a particle size of 20 to 500 microns which is administered in the manner in which snuff is taken i.e. by rapid inhalation through the nasal passage from a container of the powder held close up to the nose.

In addition to the aforementioned ingredients, the formulations of this invention may include one or more additional ingredients such as diluents, buffers, flavouring agents, binders, surface active agents, thickeners, lubricants, preservatives eg. methylhydroxybenzoate (including anti-oxidants), emulsifying agents and the like. Any other therapeutic ingredient may comprise one or more of the following: antibiotic (e.g. anti-bacterial), anti-fungal and anti-viral agents, and anti-histamines (particularly peripherally acting ant-histamines). However, when such other agent(s) are also present, according to another aspect of the invention, the compound of formula (I) or physiologically acceptable salt thereof and the other agent(s), need not necessarily be present as a pharmaceutical formulation as hereinbefore defined, but merely in combination or intimate admixture, i.e. optionally, a pharmaceutically acceptable carrier need not be present.

The combination with anti-histamines is particularly favoured for antiasthmatic use. Such an anti-histamine may be selected from any compound described in European Patent Applications EP 0 859 959 A and EP O 117 302 A. The amount and dosage regime for such an anti-histamine may be chosen from any of those recited in the latter two European Specifications. Especially preferred are the anti-histamines (E)-3-(6-(3-pyrrolidino-1-(4-tolyl)prop-1E-enyl(-2-pyridyl)acrylic acid and (E)-3-(6-(3-pyrrolidino-1-(4-tolyl)prop-1E-enyl(-2-pyridyl)propionic acid. Another preferred anti-histamine is (E)-1-(4-methylphenyl)-1-(2-pyridyl)-3-pyrrolidinoprop-1-ene, otherwise known as triprolidine.

For delaying senescence of cut or picked plant matter, or for controlling plant growth, the compounds of formula (I) and their salts are preferably presented in a suitable composition, optionally containing one or more other agents for enhancing the freshness of the plants. Such compositions include solutions and suspensions of the compound in a suitable medium such as an aqueous medium.

The compositions may be applied by immersing part (eg the cut end) or whole of the plant or by spraying the plants before ar after cutting or picking, or by application to the root structure before or after picking. The compounds may also be applied by being spread on the soil prior to cutting or picking and conveyed to the plant roots by rainwater, or by other watering means.

When applied in aqueous solution, the compounds may be presented in a concentration of from 1$\mu$M to 1M, for example 100$\mu$M to 100mM. A typical concentration might be about 1mM.

The compounds of formula (I) and their salts may be prepared by the following process which (subject to any provisos expressed herein) constitutes a further aspect of the present invention:-

a) for the preparation of compounds of formula (I) in which Q represents a non-cyclic moiety (as hereinbefore defined) in which $R^1$ represents hydrogen, reacting a compound of formula (II)

$R^6NHOH$ (II)

with a compound of formula (III)

$R^7$-$R^8$ (III)

(in which one of $R^6$ and $R^7$ is a group -$COR^2$ wherein $R^2$ is as hereinbefore defined and the other is a group of formula (Ar-(L-Ar')$_q$-(X)$_k$-(Y)$_p$-as hereinbefore defined, and $R^8$ is a group capable of reacting with the NH group in the compound of formula (II) thereby to bring about formation of the corresponding hydroxamic acid or derivative thereof); or

b) for the preparation of compounds of formula (I) in which $R^1$ represents hydrogen, reacting a compound of formula (V)

$R^9N(OZ^1)H$ (V)

(wherein $R^9$ is a group -$COR^2$ wherein $R^2$ is as hereinbefore defined or a group of formula Ar-(L-Ar')$_q$-(X)-$_k$-(Y)$_p$-as hereinbefore defined, as appropriate, and $Z^1$ is hydrogen or an appropriate protecting group) with an appropriate acylating agent, and where $Z^1$ is a protecting group, subjecting the reaction product to such conditions and/or reacting with one or more reagents as appropriate to effect removal of said

protecting group;

and optionally if desired, effecting one or more of the following interconversions in any desired order:-

(i) when in the compound of formula (I) so formed, any of $R^1$, $R^2$ and (in the definition of $R^1$) $R^4$ and $R^5$ are hydrogen atoms, converting said compound to a corresponding compound of formula (I) wherein any of said hydrogen atoms as desired, are converted to $C_{1-4}$ alkyl groups;

(ii) converting the compound of formula (I) to a corresponding salt thereof;

(iii) when in the compound of formula (I) so formed, n is 0, m is 1 and $R^1$ is hydrogen, converting said compound to a corresponding compound of formula (I) wherein $R^1$ is a group of formula $-COR^3$ as hereinbefore defined;

(iv) when in a compound of formula (I), $R^1$ is a group of formula $-COR^3$ in which $R^3$ is a $C_{1-4}$ alkyl group substituted by carboxy, converting said compound to a corresponding compound in which $R^3$ is $C_{1-4}$ alkyl substituted by $C_{1-4}$ alkoxycarbonyl.

In process option (a) above, the compound of formula (II) may be used in the form of a salt thereof and the compound of formula (III) for example is an appropriate mixed anhydride or activated acid such as an acid halide (e.g. the chloride). Preferably, the reaction is effected in a suitable solvent and where the compound of formula (II) is in the form of a salt, in the presence of a base, such as an appropriate amine, to liberate the free hydroxylamine compound in situ.

In process option (b), where the group $Z^1$ in the compound of formula (V) is a protecting group, this may for example be selected from and acetyl, benzyl, O-benzyl, trityl, tetrahydropyranyl, O-tetrahydropyranyl, O-t-butyl and benzoyl. The protecting group may be removed by treatment with acid or base or by hydrogenation as will readily be apparent to those skilled in the art. In general, suitable protecting groups and methods for their removal will be found in T.W. Greene, Protective Groups in Organic Synthesis, Wiley, New York, 1981. Particular examples of removal of such leaving groups include removal of an O-benzyl group by hydrogenolysis over 5% palladium on charcoal at room temperature, or removal of O-tetrahydropyranyl with pyridinium para-toluene sulphate in refluxing methanol.

When process option (b) is a used to prepare a compound of formula (I) wherein $R^2$ is an amino group or a mono-amine derivative and $Z^1$ is a protecting group the acylating agent may be an isocyanate of formula (VII).

$Q^1NCO$ (VII)

(wherein $Q^1$ is said $R^2$ group minus the -NH portion) and the reaction is effected in a suitable solvent such as toluene, optionally at a temperature above ambient.

When process option (b) is used to prepare a compound of formula (I) in which $R^2$ is other than as specified in the preceding paragraph (whether Z is hydrogen or a protecting group), the acylating agent may for example be an appropriate mixed anhydride or activated acid, such as an acid halide (for example chloride),

Optional conversion (i) may for example be effected by reaction with an appropriate alkyl halide or sulphate in the presence of a mild base. Where one or more but not all of a number of hydrogen atoms are to be selectively alkylated, the conversation may require one or more steps of protection and subsequent deprotection.

Optional conversion (ii) conveniently may be effected by reaction with an appropriate organic or mineral acid, or with a base.

Optional conversion (iii) when used for the preparation of compounds wherein $R^3$ is a group of formula $-N(R^4)R^5$, may comprise reacting the compound of formula (I) with an appropriate isocyanate, suitably in an appropriate solvent such as tetrahydrofuran or toluene, optionally in the presence of a catalyst such as 1,8-diazabicyclo [5.4.0]undec-7-ene.

Optional conversion (iii) when used for the preparation of compounds wherein $R^3$ is an alkyl group optionally substituted by a carboxy group or a $C_{1-4}$ alkoxycarbonyl group, may comprise reacting the compound of formula (I) with an appropriate acylating agent such as a mixed anhydride or a suitable activated acid, for example an acid halide such as a chloride. Preferably, the reaction is effected in a solvent such as methylene dichloride or tetrahydrofuran, suitably at from around 0° to ambient temperature.

Optional conversation (iv) may be effected by reaction with the appropriate alcohol in the presence of a suitable mineral acid such as sulphuric.

Salts derived from acids include the acetate, adipate, alginate, aspartate, benzoate, benzenesulphonate, bisulphate, butyrate, citrate, camphorate, camphorsulphonate, cyclopentanepropionate, digluconate, dodecylsulphate, ethanesulphonate, fumarate, glucoheptanoate, glycerophos-phate, hemisulphate, heptanoate, hexanoate, hydrochloride, hydrobromide, hydroiodide, 2-hydroxyethanesulphonate, lactate, maleate,

methanesulphonate, 2-naphthalenesulphonate, nicotinate, oxalate, palmoate, pectinate, persulphate, 3-phenyl-proprionate, picrate, pivalate, proprionate, succinate, tartrate, thiocyanate, tosylate, and undecanoate.

Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine and lysine.

Quaternary ammonium salts can be formed where a nitrogen-containing group is present, for example by reaction with lower alkyl halides, such as methyl, ethyl, propyl, and butyl chlorides, bromides and iodides; dialkyl sulphates, long chain halides such as decyl, lauryl, myristyl and stearyl chlorides, bromides and iodides, aralkyl halides like benzyl and phenethyl bromides.

Thus, subject to the requirements of novelty and inventive step, according to the present invention we may claim (inter alia):-

(a) a compound of formula (I) or an acid addition salt thereof;

(b) a method for preparing a compound of formula (I) or a pharmacologically acceptable acid addition salt thereof;

(c) a pharmaceutical formulation comprising a compound of formula (I) or a physiologically acceptable salt thereof and a pharmaceutically acceptable carrier therefor;

(d) a method for preparing such formulations;

(e) a method for the inhibition of the lipoxygenase and/or cyclooxygenase pathways of the arachidonic acid metabolism by use of a non-toxic, effective, inhibitory amount of a compound of formula (I) or a physiologically acceptable salt thereof;

(f) a method for the prophylaxis or treatment of disease in a mammal, including man, comprising the administration to said mammal of a non-toxic, therapeutically or prophylactically effective amount of a compound of formula (I) or a physiologically acceptable salt thereof;

(g) a method for the prophylaxis or treatment of any individual condition described herein, in a mammal, including man, comprising the administration to said mammal of a non-toxic therapeutically or prophylactically effective amount of a compound of formula (I) or a physiologically acceptable salt thereof;

(h) a method for the prophylaxis or treatment of asthma in a mammal, including man, comprising administration to said mammal of a non-toxic, effective, anti-asthmatic amount of a compound of formula (I) or a physiologically acceptable salt thereof;

(i) a compound of formula (I) or a physiologically acceptable salt thereof for use in medicine, especially as defined in (f)-(h) above.

(j) use of a compound of formula (I) or a physiologically acceptable salt thereof in the manufacture of medical therapeutic agents, particularly those for use as defined in (f)-(h) above;

(k) a method of regulating the growth of, or delaying senescence in vegetable matter by application to said matter of an effective amount of a compound of formula (I) or a salt thereof; or

(l) any novel feature described herein.

The following Examples are provided by way of an illustration of the present invention and should in no way be construed as a limitation thereof. All temperatures indicated are in degrees Celsius.

## Examples

### Example 1

### Preparation of N-(4-Benzyloxybenzyl)acetohydroxamic Acid

Sodium cyanoborohydride (21.3g) was added in portions to a solution of 4-benzyloxybenzaldehyde oxime (51g) in acetic acid (250ml) at ca 50° (cooling). After the reduction was complete, acetic anhydride (22.5ml) was added in one portion, and the mixture was stirred for 1 hour. The mixture was then poured into water, and the neutral product was isolated with ethyl acetate. The residue was treated with potassium carbonate (2g) in methanol (400ml) to hydrolyse the O-acetyl material, then the solvent was evaporated. Addition of 20% citric acid solution (400ml) and isolation with ethyl acetate furnished N-(4-Benzyloxybenzyl) acetohydroxamic acid (32g), m.pt. 119-120° [from ethyl acetate-light petroleum (b.pt.60-80°)].

### Example 2

### Preparation of N-[4-(2-Pyridyloxy)benzyl]acetohydroxamic Acid

2-Bromopyridine (4.74g) was added to the sodium salt of 4-methylphenol [from 50% sodium hydride

(1.58g) and the phenol (3.56g)] in dimethyl sulphoxide, and the mixture was stirred for 20 hours at 150 ° C. Addition of water, and isolation of non-phenolic material with ether gave the pyridyloxy compound (4.81g). Without purification, this compound (4.74g) was refluxed in carbon tetracholoride (125ml) with N-bromosuccinimide (5.02g) and azo-bisisobutyronitrile initiator (50mg). After 1 hour (more initiator added after 15 and 30 minutes), the filtered solution was evaporated in vacuo. The crude product (6.76g) was immediately stirred with O-(tetrahydropyran-2-yl)hydroxylamine (8.99g) in N, N-dimethylformamide (65ml) for 65 hours at room temperature. Addition of water and isolation with ether provided the protected hydroxylamine as a viscous oil. Acetylation of the crude protected hydroxylamine (3.84g) in methylene dichloride (30ml) was effected with acetic anhydride (1.44g) for 2 hours at room temperature. Evaporation of solvent, and isolation of non-acidic material with ether furnished the O-protected hydroxamic acid, which was purified by chromatography over silica gel [elution with 1:1 ethyl acetate/light petroleum (b.pt. 60-80 ° )](2.61g).

A mixture of the O-tetrahydropyranyl hydroxamic acid (2.6g) and pyridinium p-toluenesulphonate (191mg) in methanol (25ml) was refluxed for 9 hours, then evaporated in vacuo. Isolation with ethyl acetate afforded N-[4-(2-pyridyloxy)benzyl]acetohydroxamic acid (1.42g), m.pt. 97-99 ° C after recrystallization from ethyl acetate-light petroleum (b.pt. 60-80 ° ).

Examples 3-40

The following compounds were prepared in a manner generally analogous to the method of Examples 1 and 2:-

3) N-[2-(2-Naphthyloxy)ethyl]benzylhydroxamic acid, m.pt. 163-165 ° C
4) N-[2-(5,6,7,8-Tetrahydro-2-naphthyloxy)ethyl]-acetohydroxamic acid, m.pt. 73-74 ° C
5) N-[1-(4-Biphenylyl)ethyl]acetohydroxamic acid, m.pt. 143-153 ° C, vague m.pt.
6) N-[1-(4-Benzyloxy-2-hydroxyphenyl)ethyl]acetohydroxamic acid, m.pt. 149-150 ° C
7) N-(2-Benzyloxybenzyl)acetohydroxamic acid, oil
8) N-(3-Benzyloxybenzyl)acetohydroxamic acid, m.pt. 95-98 ° C
9) N-(3-Phenoxylbenzyl)acetohydroxamic acid, m.pt. 81-82 ° C
10) N-(4-Biphenylylmethyl)acetohydroxamic acid, m.pt. 152-155 ° C
11) N-[4-(1-Naphthylmethoxy)benzyl]acetohydroxamic acid, m.pt. 127-130 ° C
12) N-[4-(2-Naphthylmethoxy)benzyl]acetohydroxamic acid, m.pt. 161-14 ° C
13) N-(4-Phenoxybenzyl)acetohydroxamic acid, m.pt. 116-119 ° C
14) N-(4-Benzyloxybenzyl)pivalohydroxamic acid, m.pt. 143-144 ° C
15) N-(4-Benzyloxybenzyl)-2-methylpropanohydroxamic acid, m.pt. 113-115 ° C
16) N-(4-Phenylcarbamoylbenzyl)acetohydroxamic acid, m.pt. 194-196 ° C
17) N-[(2',4'-Difluoro-4-biphenylyl)methyl]acetohydroxamic acid, m.pt. 134-135 ° C
18) N-[1-(2',4'-Difluoro-4-biphenylyl)ethyl]-2,2-dimethylpropanohydroxamic acid, m.pt. 152-153 ° C
19) N-[4-(4-Biphenylylmethoxy)benzyl]acetohydroxamic acid, m.pt. vague, softens 165 ° C melts 175 ° C
20) N-[4-(2,4-Difluorobenzyloxy)benzyl]acetohydroxamic acid, m.pt. 113-115 ° C
21) N-[4-(2,4-Difluorobenzyloxy)benzyl]pivalohydroxamic acid, m.pt. 134-136 ° C
22) N-[5,6,7,8-Tetrahydro-2-naphthyl)methyl]acetohydroxamic acid, m.pt. 79-81 ° C
23) N-[2-(5,6,7,8-Tetrohydro-2-naphthyloxy)ethyl]-pivalohydroxamic acid, m.pt. 85-87 ° C
24) N-(5,6,7,8-Tetrahydro-2-naphthylallyl)acetohydroxamic acid, softens, 95 ° C melts, 106-107 ° C
25) N-(5,6,7,8-Tetrahydro-2-naphthylallyl)pivalohydroxamic acid, m.pt. 144-145 ° C
26) N-(5,6,7,8-Tetrahydro-2-naphthylmethyl)pivalohydroxamic acid, m.pt. 103-105 ° C
27) N-[2-(2',4'-Difluoro-4-biphenylyl)ethylacetohydroxamic acid, m.pt. 122-123 ° C
28) N-(4-Isobutylbenzyl)acetohydroxamic acid, m.pt. 89-90 ° C
29) N-[1-(4-Biphenyl)ethyl]pivalohydroxamic acid, m.pt. 170-171 ° C
30) N-[(4-Biphenylyl)methyl]pivalohydroxamic acid, m.pt. 164-165 ° C
31) N-(3-Phenoxycinnamyl)acetohydroxamic acid, light brown oil
32) N-[2-(4-Biphenylyloxy)ethyl]acetohydroxamic acid, m.pt. 125-127 ° C
33) N-[3-(4-Benzyloxyphenyl)prop-2-enyl]acetohydroxamic acid, m.pt. 148-149 ° C
34) N-[4-(2-Pyridyl)benzyl]acetohydroxamic acid, m.pt. 134-135 ° C (softens ca 125 ° C)
35) N-[4-(2,4-Difluorophenoxymethyl)benzyl]acetohydroxamic acid, m.pt. 112-113 ° C
36) N-(5-Phenoxymethyl-2-thienylmethyl)acetohydroxamic acid, m.pt. 103-104 ° C
37) N-(6-Phenoxy-2-pyridylmethyl)acetohydroxamic acid
38) N-[3-(4-Fluoro-3-phenoxyphenyl)prop-2-enyl]acetohydroxamic acid
39) N-[4-(Benzylthio)benzyl]acetohydroxamic acid
40) N-[3-(2-Pyridyloxy)benzyl]acetohydroxamic acid

Example 41

Preparation of 1-Hydroxy-1-[2-(2-naphthyloxy)ethyl]-3-phenylurea

A mixture of 2-naphthyloxyacetaldehyde hydrate (9.85g), benzyloxyamine (5.94g) and ethanol (100ml) was stirred overnight under nitrogen to precipitate 11.21g of the protected oxime, m.pt. 68-69.5°C. The oxime (10.1g) in acetic acid (100ml) was reduced with sodium cyanoborohydride, then the isolated O-benzyl hydroxylamine, without purification, was treated with phenyl isocyanate (1 equivalent) in toluene for 4 hours at 110°C. The product had m.pt. 75-77°C after chromatography over silica gel (elution with methylene chloride) and recrystallization from SVM. Hydrogenation of the last-mentioned O-benzylhydroxyurea over 5% palladium on charcoal in ethanol containing a few drops of acetic acid provided 1-Hydroxy-1-[2-(2-naphthyloxy)ethyl]-3-phenylurea, m.pt. 165-167°C (from ethanol).

Example 42

Preparation of N-(4-Benzyloxybenzyl)-N-hydroxy-N-methylurea

Sodium cyanoborohydride (1.94g) was added to 4-Benzyloxybenzaldoxime (3.5g) in acetic acid (30ml) under $N_2$, and the mixture was stirred overnight at room tempera ture. Solvent was evaporated, and the product (4g) was isolated with ether. Methyl isocyanate (0.88g) in ether (10ml) was added dropwise to a solution of the crude hydroxylamine in toluene (100ml) at 0°, and the mixture was stirred for 3 hours at room temperature to precipitate N-(4-Benzyloxybenzyl)-N-hydroxy-N'-methylurea (2.2g), m.pt. 152-154° after recrystallization from ethyl acetate-light petroleum (b.pt. 60-80°).

Examples 43-51

The following compounds were prepared in a manner generally analogous to that described in Examples 41 and 42:-

43) N-[3-(4-Fluoro-3-phenoxyphenyl)prop-2-enyl]-N-hydroxy-N'-methylurea
44) N-[3-(4-Fluoro-3-phenoxyphenyl)prop-2-enyl]-N-hydroxy-N'-t-butylurea
45) N-[3-(4-Fluoro-3-phenoxyphenyl)prop-2-enyl]-N-hydroxy-N'-cyclohexylurea
46) N-[3-(4-Fluoro-3-phenoxyphenyl)prop-2-enyl]-N-hydroxy-N'-phenylurea
47) N-(Biphenyl-4-ylmethyl)-N-hydroxy-N'-methylurea
48) N-(Biphenyl-4-ylmethyl)-N-hydroxy-N'-t-butylurea
49) N-(Biphenyl-4-ylmethyl)-N-hydroxy-N'-cyclohexylurea
50) N-(Biphenyl-4-ylmethyl)-N-hydroxy-N'-phenylurea
51) N-Hydroxy-N'-methyl-N-[3-(2-pyridyloxy)benzyl]urea

Example 52

Preparation of N-(4-Benzyloxybenzyl)-O-methylcarbamoylacetohydroxamic Acid

A mixture of N-(4-benzyloxybenzyl)acetohydroxamic acid (1.35g), methyl isocyanate (0.65g) and 1,8-diazabicyclo [5.4.0] undec-7-ene catalyst (1 drop) in tetrahydrofuran (10ml) was stirred for 3 hours then left overnight to provide N-(4-benzyloxybenzyl)-O-methylcarbamoylacetohydroxamic acid (1.35g) m.pt. 101-102° [from ethyl acetate-light petroleum (b.pt. 60-80°)].

Example 53-55

The following compounds were prepared in a manner generally analogous to that described in Example 52:-

53) N-[2-(2-Naphthylthio)ethyl-N-(phenylcarbamoyloxy)acetamide, m.pt. 96-98°C
54) N-[4-(Benzyloxybenzyl)-O-(methylcarbamoyl)pivalohydroxamic acid, m.pt. 135-136°C
55) N-(4-Benzyloxybenzyl)-O-(2,2-dimethylethyl)carbamoylacetohydroxamic acid, m.pt. l82-83°C

Example 56

Preparation of N-(4-Benzyloxybenzyl)-N-(t-butylcarbonyloxy)acetamide

Pivaloyl chloride (1.36ml) was added dropwise to a solution of N-(4-benzyloxybenzyl) acetohydroxamic acid (2.71g) and triethylamine (1.7ml) in methylene dichloride (15ml) and dimethylaminopyridine (100ml). The mixture was stirred for 2 hours at room temperature, then neutral material was isolated with ether. The product, N-(4-Benzyloxybenzyl)-N-(t-butylcarbonyloxy)acetamide, was a colourless oil.

Example 57

3-[N-(4-Benzyloxybenzyl)acetamidooxycarbonyl]propanoic acid, m.pt. 102-105° was prepared in a manner generally analogous to that described in Example 56.

Example 58

Preservation of Cut Flowers

The compound of Example 1 was made up as a 1 mM solution and the cut stem ends of cut carnations were immersed in the resultant solution in order to prolong their freshness.

Pharmaceutical Formulations

In the following formulation Examples, the "Active Ingredient" may be any compound of formula (I) or a physiologically acceptable salt thereof, for example the compound of Example 1.

Example A: Tablet:

| | In one tablet |
|---|---|
| Active Ingredient | 5.0 mg |
| Lactose | 82.0 mg |
| Starch | 10.0 mg |
| Povidone | 2.0 mg |
| Magnesium Stearate | 1.0 mg |

Mix together the active ingredient, lactose and starch. Granulate the powders using a solution of povidone in purified water. Dry the granules, add the magnesium stearate and compress to produce tablets, 100mg per tablet.

Example B: Ointment

| | |
|---|---|
| Active Ingredient | 1.0 g |
| White Soft Paraffin | to 100.0 g |

Disperse the active ingredient in a small volume of the vehicle. Gradually incorporate this into the bulk to produce a smooth, homogeneous product.Fill into collapsible metal tubes.

Example C: Cream for Topical Use

| | |
|---|---|
| Active Ingredient | 1.0 g |
| Polawax GP 200 | 20.0 g |
| Lanolin Anhydrous | 2.0 g |
| White Beeswax | 2.5 g |
| Methyl Hydroxybenzoate | 0.1 g |
| Distilled Water | to 100.0 g |

Heat the Polawax, beeswax and lanolin together at 60 ° C. Add a solution of methyl hydroxybenzoate. Homogenise using high speed stirring. Allow the temperature to fall to 50 °. Add and disperse the active ingredient. Allow to cool with slow speed stirring.

Example D: Lotion for Topical Use

| | |
|---|---|
| Active Ingredient | 1.0 g |
| Sorbitan Monolaurate | 0.6 g |
| Polysorbate 20 | 0.6 g |
| Cetostearyl Alcohol | 1.2 g |
| Glycerin | 6.0 g |
| Methyl Hydroxybenzoate | 0.2 g |
| Purified Water B.P. | to 100.00 ml |

The methyl hydroxybenzoate and glycerin were dissolved in 70ml of the water at 75 ° C. The sorbitan monolaurate, Polysorbate 20 and cetostearyl alcohol were melted together at 75 ° C and added to the aqueous solution. The resulting emulsion was homogenised, alowed to cool with continuous stirring and the active ingredient added as a suspension in the remaining water. The whole was stirred until homogeneous.

Example E: Eye Drops

| | |
|---|---|
| Active Ingredient | 0.5 g |
| Methyl Hydroxybenzoate | 0.01 g |
| Propyl Hydroxybenzoate | 0.04 g |
| Purified Water B.P. | to 100.00 ml |

The methyl and propyl hydroxybenzoates were dissolved in 70ml purified water at 75 ° and the resulting solution then allowed to cool. The active ingredient was added next and the solution made up to 100ml with purified water. The solution was sterilised by filtration through a membrane filter 0.22 μ pore size and packed aseptically into suitable sterile containers.

Example F: Injection Solution

| | |
|---|---|
| Active Ingredient | 10.0 mg |
| Water for Injections B.P. | to 1.0 ml |

The active ingredient was dissolved in half of the Water for Injections and then made up to volume and sterilised by filtration. The resulting solution was distributed into ampoules under asceptic conditions.

Pulmonary Formulations

In formulations G and H below, the "Active Ingredient" may be any compound of formula (I) or physiologically acceptable salt thereof.

Example G: Powder Capsules for Inhalation

| | |
|---|---|
| Active Ingredient (0.5-7.0 μm powder) | 4 mg |
| Lactose (30-90 μm powder) | 46.0 mg |

The powders were mixed until homogeneous and filled into suitable sized hard gelatin capsules, 50 mg of mixture per capsule.

Example H: Inhalation Aerosol

| | |
|---|---|
| Active Ingredient (0.5-7.0 μm powder) | 200 mg |
| Sorbitan Trioleate | 100 mg |
| Saccharin Sodium (0.5-7.0 μm powder) | 5 mg |
| Methanol | 2 mg |
| Trichlorofluoromethane | 4.2 g |
| Dichlorodifluoromethane to | 10.0 ml |

The Sorbitan Trioleate and Menthol were dissolved in the Trichlorofluoromethane. The Saccharin Sodium and active ingredient were dispersed in the mixture which was then transferred to a suitable aerosol canister and the Dichlorofluoromethane injected through the valve system. This composition provides 2 mg of active ingredient in each 100 μl dose.

In vitro inhibition of 5-lipoxygenase (LO) and Cyclooxygenase (CO)

Blood from normal aspirin-free volunteers was centrifuged to separate leukocytes from red cells and platelets. The leukocytes were homogenised and 5μ M arachidonic acid added, followed by incubation at 37° for 5 minutes. The reaction was stopped by boiling. Radioimmunassay was conducted for leukotriene $B_4$ (an LO product) and thromboxane $B_4$ (a CO product). Results were calculated as $IC_{50}$μ M activity against each enzyme.

**Claims**

**1.** Any novel compound of formula (I)

```
                        OR¹
                       /
Ar-(L-Ar')q-(X)k-(Y)pN                    (I)
                       \
                        COR²
```

wherein:-

k, p and q are independently 0 or 1, provided that when k is 1 then p must also be 1;

Ar represents either:

(i) naphthyl, tetrahydronaphthyl or pyridyl, any of which is optionally substituted by one or more substituents independently selected from $C_{1-4}$ alkyl (which may itself optionally be substituted by one or more halogen atoms), $C_{1-4}$ alkoxy, halo, nitro, amino, carboxy, $C_{1-4}$ alkoxycarbonyl and hydroxy, or

(ii) phenyl optionally substituted by one or more substituents independently selected from phenyl (optionally substituted by one or more substituents independently selected from those specified as optional substituents in (i) above) and said optional substituents specified in (i) above;

L is selected from $-(CH_2)_r-$ (where r is 1-4), -O-, $-CH_2O-$, $-CH_2S-$, - $OCH_2-$, -CONH-, -NHCO-, -CO- and $-CH_2NH-$, and,

Ar' represents phenylene, thienylene or pyridylene, any of which may be optionally substituted by one or more substituents independently selected from those specified as optional substituents in definition (i) of Ar;

X represents oxygen, sulphur or carbonyl, provided that at least one atom separates said carbonyl group from any carbonyl group in Q as defined below;

Y is $C_{1-10}$ alkylene or $C_{2-10}$ alkenylene;

$R^1$ is independently selected from hydrogen, $C_{1-4}$ alkyl, groups as defined for Ar above and groups of formula $-COR^3$ in which $R^3$ is selected from $C_{1-4}$ alkyl (optionally substituted by a carboxy or $C_{1-4}$ alkoxycarbonyl group) and groups of formula $-N(R^4)R^5$ in which $R^4$ is hydrogen or $C_{1-4}$ alkyl and $R^5$ represents hydrogen $C_{1-4}$ alkyl or phenyl optionally substituted by one or more substituents independently selected from those specified as optional substituents in the definition (i) of Ar,

and $R^2$ is selected from hydrogen, $C_{1-4}$ alkyl, amino, $C_{1-4}$ alkylamino, di-$C_{1-4}$ alkylamino, $C_{5-7}$ cycloalkylamino, $C_{5-7}$ cycloalkyl ($C_{1-4}$ alkyl) amino, anilino, N-$C_{1-4}$ alkylanilino and groups as defined for Ar above;

and salts thereof;

with the proviso that:-

when q is 0, k is 0 or 1 and p is 1, Ar is phenyl or naphthyl, either being optionally substituted by one or more substituents as specified in definition (i) of Ar, and X is oxygen or sulphur (in the case when k is 1) Y is $C_{1-10}$ alkylene and one of $R^1$ and $R^2$ is hydrogen or $C_{1-4}$ alkyl;

then the other of $R^1$ and $R^2$ is neither hydrogen nor $C_{1-4}$ alkyl.

**2.** A compound or salt as claimed in claim 1, with the further proviso that

when $R^1$ is hydrogen or $C_{1-4}$ alkyl and $R^2$ is phenyl optionally substituted by a single substituent selected from $C_{1-4}$ alkyl, $C_{1-4}$ alkoxy and phenyl optionally substituted by one or more substituents independently selected from these specified as optional substituents in definition (i) of Ar,

then at least one of k and p must be 1, and in the case when k is 0 and p is 1, Y must be $C_{1-10}$ alkenylene.

**3.** A compound or salt as claimed in claim 1 or 2 wherein

q is 0 and p is 1;

Ar represents naphthyl optionally substituted by one or more substituents independently selected from those defined in definition (i) of Ar;

X (in the case when k is 1) is oxygen; and

$R^2$ is $C_{1-4}$ alkyl.

**4.** A compound or salt as claimed in any of claims 1-3 wherein

Ar represents phenyl optionally substituted by one or more substituents independently selected from those defined in definition (ii) of Ar;

L is -O- or $-CH_2O-$; and

Ar' is phenylene optionally substituted by one or more substituents independently selected from those defined in definition (i) of Ar.

**5.** A compound selected from the following
N-(3-phenoxycinnamyl)acetohydroxamic acid,
N-(4-benzyloxybenzyl)acetohydroxamic acid,
N-[2-(5,6,7,8-tetrahydro-2-naphthyloxy)ethyl]acetohydroxamic acid
N-(5,6,7,8-Tetrahydro-2-naphthylallyl)acetohydroxamic acid
and salts thereof

**6.** A pharmaceutical formulation comprising a compound as claimed in claim 3 when dependent on claim 2 or a physiologically acceptable salt thereof, optionally one or more other pharmacologically active agents, and a pharmaceutically acceptable carrier therefor.

**7.** A process for the preparation of a compound of formula (I) as defined in claim 1 or a salt thereof, comprising:-

a) for the preparation of compounds of formula (I) in which $R^1$ represents hydrogen, reacting a compound of formula (II)

$R^6NHOH$ (II)

with a compound of formula (III)

$R^7$-$R^8$ (III)

(in which one of $R^6$ and $R^7$ is a group $R^2$ as defined in claim 1 and the other is a group of formula Ar-(L-Ar')$_q$-(X)$_k$-(Y)$_p$- as definedin claim 1, and $R^8$ is a group capable of reacting with the NH group in the compound of formula (II) thereby to bring about formation of the corresponding hydroxamic acid or derivative thereof); or

b) for the preparation of compounds of formula (I) in which $R^1$ represents hydrogen, reacting a compound of formula (V)

$R^9N(OZ^1)H$ (V)

(wherein $R^9$ is a group $R^2$ as defined in claim 1 or a group of formula Ar-(L-Ar')$_q$-(X)$_k$-(Y)$_p$- as defined in claim 1, as appropriate, and $Z^1$ is hydrogen or an appropriate protecting group) with an appropriate acylating agent, and where $Z^1$ is a protecting group, subjecting the reaction product to such conditions and/or reacting with one or more reagents as appropriate to effect removal of said protecting group;

and optionally if desired, effecting one or more of the following inter-conversions in any desired order:-

(i) when in the compound of formula (I) so formed, any of $R^1$, $R^2$ and (in the definition of $R^1$) $R^4$ and $R^5$ are hydrogen atoms, converting said compound to a corresponding compound of formula (I) wherein any of said hydrogen atoms as desired, are converted to $C_{1-4}$ alkyl groups;

(ii) converting the compound of formula (I) to a corresponding salt thereof;

(iii) when in the compound of formula (I) so formed, $R^1$ is hydrogen, converting said compound to a corresponding compound of formula (I) wherein $R^1$ is a group of formula $-COR^3$ as defined in claim 1;

(iv) when in a compound of formula (I), $R^1$ is a group of formula $-COR^3$ in which $R^3$ is a $C_{1-4}$ alkyl group substituted by carboxy, converting said compound to a corresponding compound in which $R^3$ is $C_{1-4}$ alkyl substituted by $C_{1-4}$ alkoxycarbonyl.

**8.** A compound or physiologically acceptable salt as claimed in claim 2, for use in medical therapeutic treatment or prophylaxis.

**9.** Use of a compound or physiologically acceptable salt as claimed in claim 2 in the manufacture of anti-spasmogenic agents.

**10.** Use as claimed in claim 9 wherein the agents are anti-asthmatic.

**11.** Use of a compound or physiologically acceptable salt as claimed claim 1 in the manufacture of anti-inflammatory, analgesic or anti-pyretic agents.

**Patentansprüche**

**1.** Neue Verbindung der Formel (I)

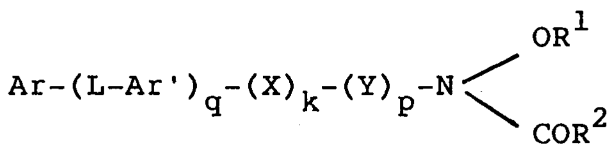

(I)

worin gilt:
k, p und q sind, unabhängig voneinander, 0 oder 1, mit der Maßgabe, daß, wenn k 1 ist, p ebenfalls 1 sein muß;
Ar bedeutet entweder:
(i) Naphthyl, Tetrahydronaphthyl ode Pyridyl, von denen eine jede Gruppe durch einen oder mehrere Substituenten, unabhängig voneinander, gegebenenfalls substituiert ist, ausgewählt aus $C_{1-4}$-Alkyl (das selbst durch ein oder mehrere Halogenatome ggf. substituiert sein kann), $C_{1-4}$-Alkoxy, Halogen, eine Nitro-, Amino-, Carboxy-, $C_{1-4}$-Alkoxycarbonyl- und Hydroxygruppe, oder
(ii) Phenyl, ggf. substituiert, unabhängig voneinander, durch einen oder mehrere Substituenten, ausgewählt aus Phenyl (ggf. substituiert, unabhängig voneinander, durch einen oder mehrere Substituenten, ausgewählt aus den oben unter (i) als ggf. vorhanden angegebenen Substituenten) und aus den genannten ggf. vorhandenen Substituenten, die oben unter (i) angegeben sind;
L ist ausgewählt aus $-(CH_2)_r-$ (worin r 1 bis 4 ist, -O-, $-CH_2O-$, $-CH_2S-$, $-OCH_2-$, -CONH-, -NHCO-, -CO- und $-CH_2NH-$, und
Ar' bedeutet Phenylen, Thienylen oder Pyridylen, von denen eine jede Gruppe durch einen oder mehrere Substituenten, unabhängig voneinander, ggf. substituiert sein kann, ausgewählt aus denjenigen, die in der Definition (i) von Ar als ggf. vorhandene Substituenten angegeben sind;
X bedeutet Sauerstoff, Schwefel oder Carbonyl;
Y ist $C_{1-10}$-Alkylen oder $C_{2-10}$-Alkenylen;
$R^1$ ist ausgewählt, unabhängig voneinander, aus Wasserstoff, $C_{1-4}$-Alkyl, Gruppen gemäß obiger Definition für Ar und Gruppen der Formel $-COR^3$, worin $R^3$ ausgewählt ist aus $C_{1-4}$-Alkyl (ggf. substituiert durch eine Carboxy- oder $C_{1-4}$-Alkoxycarbonylgruppe) und aus Gruppen der Formel $-N(R^4)R^5$, worin $R^4$ Wasserstoff oder $C_{1-4}$-Alkyl ist und $R^5$ Wasserstoff, $C_{1-4}$-Alkyl oder Phenyl darstellt, ggf. substituiert, unabhängig voneinander, durch einen oder mehrere Substituenten, ausgewählt aus den in der Definition (i) von Ar als ggf. vorhanden angegebenen Substituenten,
und $R^2$ ist ausgewählt aus Wasserstoff, $C_{1-4}$-Alkyl-, Amino-, $C_{1-4}$-Alkylamino-, Di-$C_{1-4}$-alkylamino-,$C_{5-7}$-Cycloalkylamino-, $C_{5-7}$-Cycloalkyl($C_{1-4}$alkyl)amino-, Anilino-, N-$C_{1-4}$-Alkylanilinogruppen und Gurppen gemäß obiger Definition für Ar;
sowie Salze davon;
mit der Maßgabe, daß:
<u>wenn</u> q 0, k 0 oder 1 und p 1 sind, Ar Phenyl oder Naphthyl ist, wobei jedes der beiden ggf. durch einen oder mehrere in der Definition (i) von Ar angegebene Substituenten substituiert ist, und X im Falle von k = 1 Sauerstoff oder Schwefel ist, Y $C_{1-10}$-Alkylen ist und eines von $R^1$ und $R^2$ Wasserstoff oder $C_{1-4}$-Alkyl ist,
<u>dann</u> das andere von $R^1$ und $R^2$ weder Wasserstoff noch $C_{1-4}$-Alkyl ist.

**2.** Verbindung oder Salz gemäß Anspruch 1, mit der weiteren Maßgabe, daß
wenn $R^1$ Wasserstoff oder eine $C_{1-4}$-Alkylgruppe ist und $R^2$ eine Phenylgruppe darstellt, die gegebenenfalls durch einen einzelnen Substituenten substituiert ist, ausgewählt aus einer $C_{1-4}$-Alkyl, $C_{1-4}$-Alkoxy- und Phenylgruppe, die gegebenenfalls durch einen oder mehrere Substituenten, unabhängig voneinander, substituiert ist, ausgewählt aus den in der Definition (i) von Ar als gegebenenfalls vorliegend angegebenen Substituenten,
dann mindestens eines von k und p 1 sein muß, und im Fall k = 0 und p = 1 Y eine $C_{2-10}$-Alkenylengruppe sein muß.

**3.** Verbindung oder Salz gemäß Anspruch 1 oder 2, worin
q 0 oder p 1 sind;
Ar eine Naphthylgruppe darstellt, die gegebenenfalls durch einen oder mehrere Substituenten, unabhängig voneinander, substituiert ist, ausgewählt aus den in Definition (i) von Ar definierten;
X (im Fall k = 1) Sauerstoff ist; und
$R^2$ eine $C_{1-4}$-Alkylgruppe ist.

**4.** Verbindung oder Salz gemäß jedem der Ansprüche 1 bis 3, worin
Ar eine Phenylgruppe darstellt, die gegebenenfalls durch einen oder mehrere Substituenten, unabhängig voneinander, substituiert ist, ausgewählt aus den in Definition (ii) von Ar definierten;
L -O- oder $-CH_2O-$ ist; und
Ar' eine Phenylengruppe darstellt, die gegebenenfalls durch einen oder mehrere Substituenten, unabhängig voneinander, substituiert ist, ausgewählt aus den in Definition (i) von Ar definierten.

**5.** Verbindung, ausgewählt aus
N-(3-Phenoxycinnamyl)acetohydroxamsäure,
N-(4-Benzyloxybenzyl)acetohydroxamsäure,
N-[2-(5,6,7,8-Tetrahydro-2-naphthyloxy)ethyl]acetohydroxamsäure,
N-(5,6,7,8-Tetrahydro-2-naphthylallyl)acetohydroxamsäure sowie Salze davon.

**6.** Pharmazeutische Formulierung, enthaltend eine Verbindung gemäß Anspruch 3, wenn dieser von Anspruch 2 abhängt, oder ein physiologisch verträgliches Salz davon, gegebenenfalls einen oder mehrere weitere pharmakologisch wirksame Mittel sowie einen pharmazeutisch verträglichen Träger dafür.

**7.** Verfahren zur Herstellung einer Verbindung der Formel (I) gemäß Anspruch 1 oder eines Salzes davon, wobei man:

a) zur Herstellung von Verbindungen der Formel (I), worin $R^1$ Wasserstoff darstellt, eine Verbindung der Formel (II)

$R^6NHOH$ (II)

mit einer Verbindung der Formel (III) reagieren läßt

$R^7$-$R^8$ (III)

(worin der eine Rest von $R^6$ und $R^7$ eine $-COR^2$-Gruppe, worin $R^2$ wie in Anspruch 1 definiert ist, und der andere Rest eine Gruppe der in Anspruch 1 definierten Formel Ar-(L-Ar')$_q$-(X)$_k$-(Y)$_p$- und $R^8$ eine Gruppe sind, die dazu befähigt ist, mit der NH-Gruppe in der Verbindung der Formel (II) zu reagieren, um dadurch die entsprechende Hydroxamsäure oder ein Derivat davon zu bilden); oder

b) zur Herstellung von Verbindungen der Formel (I), worin $R^1$ Wasserstoff darstellt, eine Verbindung der Formel (V)

$R^9N(OZ^1)H$ (V)

(worin $R^9$ eine $-COR^2$-Gruppe, worin $R^2$ wie vorstehend definiert ist, oder eine Gruppe der in Anspruch 1 definierten Formel Ar-(L-Ar')$_l$-(X)$_k$-(Y)$_p$-, wie geeignet, und $Z^1$ Wasserstoff oder eine geeignete Schutzgruppe sind) mit einem geeigneten Acylierungsmittel reagieren läßt, und wenn $Z^1$ eine Schutzgruppe darstellt, man das Reaktionsprodukt solchen Bedingungen unterzieht und/oder es mit einem oder mehreren geeigneten Reagentien reagieren läßt, um die genannte Schutzgruppe zu entfernen;

und gegebenenfalls, falls gewünscht, eine oder mehrere der folgenden gegenseitigen Umwandlungsreaktionen in jeder gewünschten Reihenfolge durchführt:

(i) wenn in der so gebildeten Verbindung der Formel (I) jeder Rest von $R^1$, $R^2$ und (in der Definition von $R^1$) $R^4$ und $R^5$ Wasserstoffatome sind, überführt man die genannte Verbindung in eine entsprechende Verbindung der Formel (I), worin ein jedes der genannten Wasserstoffatome, wie gewünscht, in $C_{1-4}$-Alkylgruppen umgewandelt ist;

(ii) man überführt die Verbindung der Formel (I) in ein entsprechendes Salz davon;

(iii) wenn in der so gebildeten Verbindung der Formel (I) $R^1$ Wasserstoff ist, überführt man die genannte Verbindung in eine entsprechende Verbindung der Formel (I), worin $R^1$ eine Gruppe der in Anspruch 1 definierten Formel $-COR^3$ ist;

(iv) wenn in einer Verbindung der Formel (I) $R^1$ eine Gruppe der Formel $-COR^3$ ist, worin $R^3$ eine durch Carboxy substituierte $C_{1-4}$-Alkylgruppe ist, überführt man die genannte Verbindung in eine entsprechende Verbindung, in der $R^3$ eine durch $C_{1-4}$-Alkoxycarbonyl substituierte $C_{1-4}$-Alkylgruppe ist.

**8.** Verbindung oder physiologisch verträgliches Salz gemäß Anspruch 2 zur Verwendung in einer medizinischen therapeutischen Behandlung oder Prophylaxe.

**9.** Verwendung einer Verbindung oder eines physiologisch verträglichen Salzes gemäß Anspruch 2 zur Herstellung anti-spasmogener Mittel.

**10.** Verwendung gemäß Anspruch 9, worin die Mittel anti-Asthmamittel sind.

**11.** Verwendung einer Verbindung oder eines physiologisch verträglichen Salzes gemäß Anspruch 1 zur Herstellung entzündungshemmender, analgetischer oder anti-pyretischer Mittel.

## Revendications

**1.** Nouveau composé quelconque de formule (I) :

$$\begin{array}{ll} \qquad\qquad\qquad\qquad\qquad\quad OR^1 & \\ \qquad\qquad\qquad\qquad\qquad / & \\ Ar-(L-Ar')_q-(X)_k-(Y)_pN & \qquad (I) \\ \qquad\qquad\qquad\qquad\qquad \backslash & \\ \qquad\qquad\qquad\qquad\qquad\quad COR^2 & \end{array}$$

où :

k, p et q sont indépendamment 0 ou 1, avec la restriction que lorsque k est 1, alors p doit aussi être 1;

Ar représente soit :

(i) naphtyle, tétrahydronaphtyle ou pyridyle, dont l'un quelconque est facultativement substitué par un ou plusieurs substituants choisis indépendamment parmi $C_{1-4}$-alcoyle (qui peut lui-même être facultativement substitué par un ou plusieurs atomes d'halogène), $C_{1-4}$-alcoxy, halo, nitro, amino, carboxyle, $C_{1-4}$-alcoxycarbonyle et hydroxyle, soit

(ii) phényle facultativement substitué par un ou plusieurs substituants choisis indépendamment parmi phényle (facultativement substitué par un ou plusieurs substituants choisis indépendamment parmi ceux spécifiés comme substituants facultatifs en (i) ci-dessus) et les substituants facultatifs spécifiés en (i) ci-dessus;

L est choisi parmi $-(CH_2)_r-$ (où r est 1-4), -O-, $-CH_2O-$, $-CH_2S-$, $-OCH_2-$, -CONH-, -NHCO-, -CO- et $-CH_2NH-$, et

Ar' est phénylène, thiénylène ou pyridylène, dont l'un quelconque peut être facultativement substitué par un ou plusieurs substituants choisis indépendamment parmi ceux spécifiés comme substituants facultatifs dans la définition (i) de Ar;

X représente oxygène, soufre ou carbonyle;

Y est $C_{1-10}$-alcoylène ou $C_{2-10}$-alcénylène, et

$R^1$ est choisi indépendamment parmi hydrogène, $C_{1-4}$-alcoyle, les radicaux tels que définis à propos de Ar ci-dessus et les radicaux de formule $-COR^3$, où $R^3$ est choisi parmi $C_{1-4}$-alcoyle (facultativement substitué par un radical carboxyle ou $C_{1-4}$-alcoxycarbonyle) et les radicaux de formule $-N(R^4)R^5$, où $R^4$ est hydrogène ou $C_{1-4}$-alcoyle et $R^5$ est hydrogène, $C_{1-4}$-alcoyle ou phényle facultativement substitué par un ou plusieurs substituants choisis indépendamment parmi ceux spécifiés comme substituants facultatifs dans la définition (i) de Ar, et

$R^2$ est choisi parmi hydrogène, $C_{1-4}$-alcoyle, amino, $C_{1-4}$-alcoylamino, di-$C_{1-4}$-alcoylamino, $C_{5-7}$-cycloalcoylamino, $C_{5-7}$-cycloalcoyl($C_{1-4}$-alcoyl)amino, anilino, N-$C_{1-4}$-alcoylanilino et les radicaux tels

que définis à propos de Ar ci-dessus;

avec la restriction que :

lorsque q est 0, k est 0 ou 1 et p est 1, Ar est phényle ou naphtyle, l'un ou l'autre étant facultativement substitué par un ou plusieurs substituants tels que spécifiés dans la définition (i) de Ar, et X est oxygène ou soufre (au cas où k est 1), Y est $C_{1-10}$-alcoylène et l'un d'entre $R^1$ et $R^2$ est hydrogène ou $C_{1-4}$-alcoyle;

alors l'autre d'entre $R^1$ et $R^2$ n'est ni hydrogène ni $C_{1-4}$-alcoyle.

**2.** Composé ou sel suivant la revendication 1, avec la restriction supplémentaire que :

lorsque $R^1$ est hydrogène ou $C_{1-4}$-alcoyle et $R^2$ est phényle facultativement substitué par un substituant unique choisi par $C_{1-4}$-alcoyle, $C_{1-4}$-alcoxy et phényle facultativement substitué par un ou plusieurs substituants choisis indépendamment parmi ceux spécifiés comme substituants facultatifs dans la définition (i) de Ar;

alors au moins l'un d'entre k et p doit être 1 et au cas où k est 0 et p est 1, Y doit être $C_{2-10}$-alcénylène.

**3.** Composé ou sel suivant la revendication 1 ou 2, où

q est 0 et p est 1;

Ar représente naphtyle facultativement substitué par un ou plusieurs substituants choisis indépendamment parmi ceux spécifiés dans la définition (i) de Ar;

X (au cas où k est 1) est oxygène, et

$R^2$ est $C_{1-4}$-alcoyle.

**4.** Composé ou sel suivant l'une quelconque des revendications 1 à 3, où

Ar représente phényle facultativement substitué par un ou plusieurs substituants choisis indépendamment parmi ceux spécifiés dans la définition (ii) de Ar;

L est -O- ou $-CH_2O-$, et

Ar' est phénylène facultativement substitué par un ou plusieurs substituants choisis indépendamment parmi ceux spécifiés dans la définition (i) de Ar.

**5.** Composé choisi parmi les suivants : l'acide N-(3-phénoxycinnamyl)acétohydroxamique, l'acide N-(4-benzyloxybenzyl)acétohydroxamique, l'acide N-[2-(5,6,7,8-tétrahydro-2-napthyloxy)éthyl]-acétoxyhydroxamique et l'acide N-(5,6,7,8-tétrahydro-2-naphtylallyl)acétohydroxamique.

**6.** Composition pharmaceutique comprenant un composé suivant la revendication 3, en dépendance de la revendication 2, ou un sel physiologiquement acceptable de celui-ci facultativement avec un ou plusieurs autres agents pharmacologiquement actifs et un excipient pharmaceutiquement acceptable.

**7.** Procédé de préparation d'un composé de formule (I) tel que défini dans la revendication 1 ou d'un sel de celui-ci, qui comprend :

a) pour la préparation de composés de formule (I), où $R^1$ représente hydrogène, la réaction d'un composé de formule (II) :

$R^6NHOH$ (II)

avec un composé de formule (III) :

$R^7$-$R^8$ (III)

(où l'un d'entre $R^6$ et $R^7$ est un radical $-COR^2$, où $R^2$ est tel que défini dans la revendication 1, et l'autre est un radical de formule $Ar-(L-Ar')_q-(X)_k-(Y)_p-$ tel que défini dans la revendication 1, et $R^8$ est un radical capable de réagir avec le radical NH du composé de formule (II) de manière à causer la formation de l'acide hydroxamique correspondant ou dérivé de celui-ci); ou

b) pour la préparation de composés de formule (I), où $R^1$ représente hydrogène, la réaction d'un composé de formule (V) :

$R^9N(OZ^1)H$ (V)

(où $R^9$ est un radical -$COR^2$, où $R^2$ est tel que défini dans la revendication 1, ou un radical de formule $Ar-(L-Ar')_q-(X)_k-(Y)_p-$ tel que défini dans la revendication 1, suivant ce qui convient, et $Z^1$ est hydrogène ou un radical protecteur approprié) avec un agent d'acylation approprié, et lorsque $Z^1$ est un radical protecteur, l'exposition du produit de réaction à des conditions telles et/ou à la réaction avec un ou plusieurs réactants, suivant ce qui convient, pour provoquer l'élimination du radical protecteur;

et facultativement, si la chose est souhaitée, l'exécution d'une ou plusieurs des interconversions ci-après dans tout ordre souhaité :

(i) lorsque dans le composé de formule (I) ainsi formé, l'un quelconque d'entre $R^1$, $R^2$ et (dans la définition de $R^1$) $R^4$ et $R^5$ sont des atomes d'hydrogène, la conversion de ce composé en un composé correspondant de formule (I), où parmi ces atomes d'hydrogène, ceux quelconques qui sont souhaités, sont convertis en radicaux $C_{1-4}$-alcoyle;

(ii) la conversion du composé de formule (I) en un sel correspondant de celui-ci;

(iii) lorsque dans le composé de formule (I) ainsi formé, $R^1$ est hydrogène, la conversion de ce composé en un composé correspondant de formule (I), où $R^1$ est un radical de formule -$COR^3$ tel que défini dans la revendication 1;

(iv) lorsque dans un composé de formule (I), $R^1$ est un radical de formule -$COR^3$, où $R^3$ est un radical $C_{1-4}$-alcoyle substitué par carboxyle, la conversion de ce composé en un composé correspondant, où $R^3$ est $C_{1-4}$-alcoyle substitué par $C_{1-4}$-alcoxycarbonyle.

**8.** Composé ou sel physiologiquement acceptable suivant la revendication 2, à utiliser dans un traitement médical thérapeutique ou la prophylaxie.

**9.** Utilisation d'un composé ou sel physiologiquement acceptable suivant la revendication 2, dans la fabrication d'agents antispasmogènes.

**10.** Utilisation suivant la revendication 9, dans laquelle les agents sont antiasthmatiques.

**11.** Utilisation d'un composé ou sel physiologiquement acceptable suivant la revendication 1, dans la fabrication d'agents anti-inflammatoires, analgésiques ou antipyrétiques.